# EUROPEAN PATENT SPECIFICATION

(11) **EP 2 691 068 B1**
(45) Date of publication and mention of the grant of the patent: **06.05.2015**
(21) Application number: 12708041.4
(22) Date of filing: 13.03.2012
(51) Int. Cl.: A61K 8/35, A61K 8/37, A61K 8/40, A61K 8/86, A61Q 17/04, A61K 8/36

(54) **A SUNSCREEN COMPOSITION**
SONNENSCHUTZZUSAMMENSETZUNG
COMPOSITION D'ÉCRAN SOLAIRE

(30) Priority: 29.03.2011 IN MM09582011; 16.06.2011 EP 11170209
(43) Date of publication of application: 05.02.2014
(73) Proprietor: Unilever N.V., 3013 AL Rotterdam (NL); Unilever PLC, London, Greater London EC4Y 0DY (GB)
(72) Inventor: CHAVAN, Mohan, Vijaykumar, Mumbai 400 099 (IN); DUGGAL, Charu, Whitefield Bangalore 560 066 (IN); GAURAV, Kumar, Whitefield Bangalore 560 066 (IN); RAUT, Janhavi, Sanjay, Sharnbrook Bedfordshire MK44 1LQ (GB); VAIDYA, Ashish, Anant, Whitefield Bangalore 560 066 (IN)
(74) Representative: Corsten, Michael Allan
(86) International application number: PCT/EP2012/054342
(87) International publication number: WO 2012/130605

(56) References cited:
- WO-A1-2008/022946
- WO-A1-2009/020676
- WO-A2-2010/129318
- WO-A2-2010/129321
- WO-A2-2011/070077
- ANONYMOUS: "Emulsifiers with HLB Values", , pages 1-3, XP007918468, Retrieved from the Internet: URL:http://www.theherbarie.com/files/resou rce-center/formulating/Emulsifi ers_HLB_Values.pdf [retrieved on 2011-04-29]

## Description

### Field of the invention

The invention relates to a sunscreen composition. The invention more particularly relates to a sunscreen composition that not only provides enhanced sun protection but does that over a sustained period of time after topically applying the composition to skin.

### Background of the invention

Solar radiation includes ultraviolet (UV) radiation, wavelength of which is between 200 nm and 400 nm. Exposure of skin to UV-A (320 to 400 nm) and UV-B (290 to 320 nm) causes various problems like reddening of the skin, localized irritation, sunburn, melanoma and formation of wrinkles. UV radiation is also known to cause damage to hair. Therefore, it is desirable to protect the skin and other keratinous substrates of the human body from the harmful effects of both UV-A and UV-B radiation.

Cosmetic compositions comprising sunscreen agents are used to protect the skin against UV radiation. The most commonly used UV-A sunscreen is of the dibenzoylmethane class. They are often used along with UV-B sunscreens to get wide spectrum sunscreen protection. When dibenzoylmethane compounds are used in sunscreen compositions along with some oil-soluble organic sunscreens, it has been reported that the stability of dibenzoylmethane sunscreen is low when applied to the skin and exposed to solar radiation.

Further, to get good sun protection factor (SPF) which is a measure of the protection from solar radiation, formulators need to include high amounts of each of these UV-A (e.g. dibenzoylmethane compound) and UV-B sunscreens which further compound the problem of stability of the dibenzoylmethane class of compounds. The present inventors have found that inclusion of a non-ionic surfactant especially of specified classes along with a specific stabliser not only provides high SPF benefits with relatively low amounts of organic sunscreens but also results in longer SPF since the organic sunscreens are more stable in the compositions.

Non-ionic surfactants have been disclosed in the past for use in skin compositions. WO 2008/022946 (Unilever) discloses a photostable cosmetic composition comprising 0.1 to 10 % dibenzoylmethane or its derivative, 0.1 to 10 % by weight p-methoxycinnamic acid or its derivative, 0.5 to 8 % by weight C8 to C18 fatty alcohol ethoxylate and 0.5 to 8 % polyalkyleneglycol.

Published Indian Patent Application No. 514/MUM/2006 (Hindustan Unilever Ltd) discloses stable sunscreen compositions with novel silicone sunscreens where one of the sunscreens is an allyloxy functionalized cyanoacrylate sunscreen.

US 2009/039322 (Hallstar) discloses alkoxy polyester compounds and methods to increase the photostability of UV-degradable photoactive compounds.

WO 2009/020676 A1 discloses sunscreen compositions containing alkoxy crylenes.

While the above references are directed to improving stability of sunscreen containing compositions, the references do not disclose a sunscreen composition resulting in prolonged and enhanced SPF while using low amounts of organic sunscreens as claimed in the present invention.

It is thus an object of the present invention to obviate the drawbacks of the prior art and provide high SPF photo-protective sunscreen compositions while ensuring prolonged efficacy of the UV-A sunscreen used therein.

Another object of the present invention is to achieve the above object using relatively low amounts of sunscreen agents thereby keeping costs low.

### Summary of the Invention

The invention provides for a sunscreen composition according to claim 1.

### Detailed description of the Invention

These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

By "A Sunscreen Composition" as used herein, is meant to include a composition for topical application to sun-exposed areas of the skin and/or hair of mammals, especially humans. Such a composition may be generally classified as leave-on or rinse off, and includes any product applied to a human body for also improving appearance, cleansing, odor control or general aesthetics. It is more preferably a leave-on product. The composition of the present invention can be in the form of a liquid, lotion, cream, foam, scrub, gel, or toner, or applied with an implement or via a face mask, pad or patch. Non-limiting examples of such sunscreen compositions include leave-on skin lotions, creams, antiperspirants, deodorants, lipsticks, foundations, mascara, sunless tanners and sunscreen lotions. "Skin" as used herein is meant to include skin on the face and body (e.g. neck, chest, back, arms, underarms, hands, legs, buttocks and scalp) and especially to the sun exposed parts thereof. The composition of the invention is also of relevance to applications on any other keratinous substrates of the human body other than skin e.g. hair where products may be formulated with specific aim of providing photoprotection.

An advantage of the present invention is that the sunscreen composition is capable of providing an SPF higher than 15, more preferably higher than 16, further more preferably higher than 18, even further more preferably higher than 20, and in highly preferred aspects higher than 25. It is preferred that the composition comprises less than 10 %, preferably less than 8 %, more preferably less than 7 %, and further more preferably, less than 6 % by weight total organic sunscreens by weight of the composition.

The sunscreen composition of the invention comprises a UV-A sunscreen which is a dibenzoylmethane selected from 4-tert-butyl-4'-methoxydibenzoylmethane, 2-methyldibenzoylmethane, 4-methyl-dibenzoyl-ethane, 4-isopropyldibenzoyl-methane, 4-tert-butyldibenzoylmethane, 2,4-dimethyldibenzoylmethane, 2,5-dimethyldibenzoylmethane, 4,4'-diisopropyl-dibenzoylmethane, 2-methyl-5-isopropyl-4'-methoxydibenzoylmethane, 2-methyl-5-tert-butyl-4'-methoxydibenzoyl methane, 2,4-dimethyl-4'-methoxy dibenzoylmethane or 2,6-dimethyl-4-tert-butyl-4'-methoxy-dibenzoylmethane. The most preferred dibenzoylmethane derivative is 4-tert.-butyl-4'-methoxydibenzoylmethane. The composition of the invention preferably comprises 0.1 to 5 %, more preferably 0.2 to 5 %, further more preferably, 0.4 to 3 %, by weight dibenzoylmethane or a derivative thereof based on total weight of the composition and including all ranges subsumed therein.

The oil soluble UV-B organic sunscreen is selected from the class of cinnamic acid, salicylic acid, diphenyl acrylic acid or derivatives thereof. Illustrative non-limiting example of oil soluble UV-B sunscreens which are commercially available and useful for inclusion in the composition of the invention are Octisalate™ (octyl salicylate), Homosalate™ (3,3,5-trimethyleyclohexyl 2-hydroxybenzoate), Neo Heliopan™ (a range of organic UV filters including ethylhexyl methoxycinnamate (Neo Heliopan AV) and ethylhexyl salicylate (Neo Heliopan OS)), Octocrylene™ (2-ethylhexyl 2-cyano-3,3-diphenyl-2-propenoate), oxybenzone or Parsol MCX™ (2-ethylhexyl-4-methoxycinnamate). It is preferred that the objects of the invention of providing high SPF as well as sustained UV-A stability during use is achieved using total organic UV-B sunscreens in the range of 0.1 to 7 %, preferably from 0.5 to 6 %, more preferably from 1 to 5 %, by weight of the composition.

It is preferred that the composition of the invention is substantially free of water soluble organic sunscreens. Water soluble sunscreens may, however, be incorporated in small amounts, preferably less than 1 %, more preferably less than 0.5 %, and most preferably less than 0.1 % and optimally absent from the composition of the invention.

An important ingredient that contributes to benefits of the present invention is a non-ionic surfactant. The non-ionic surfactant for use in the composition of the present has an HLB value of at least 9.

HLB is calculated using the Griffin method wherein HLB = 20 x Mh / M wherein Mh is the molecular mass of the hydrophilic portion of the molecule and M is the molecular mass of the whole molecule, giving a result on an arbitrary scale of 0 to 20. Typical values for various surfactants are given below:
- A value <10 : Lipid soluble (water insoluble)
- A value >10 : Water soluble
- A value from 4 to 8 indicates an anti-foam ing agent
- A value from 7 to 11 indicates a W/O (water in oil) emulsifier
- A value from 12 to 16 indicates oil in water emulsion
- A value from 11 to 14 indicates a wetting agent
- A value from 12 to 15 is typical of detergents
- A value of 16 to 20 indicates a solubiliser or hydrotrope.

The non-ionic surfactant is preferably selected from the following five classes:
(a) fatty alcohol ethoxylates with saturated carbon chain and having HLB higher than 15.5; or
(b) fatty alcohol ethoxylates with unsaturated carbon chain with HLB higher than 12.
(c) alkyl phenol ethoxylates having HLB higher than 15;
(d) polyoxyethylene sorbitan alkyl esters with saturated C12 to C16 carbon chain and having HLB higher than 12;
(e) polyoxyethylene sorbitan alkyl esters with unsaturated C18 carbon chain and having HLB higher than 9;

Suitable examples of (a) fatty alcohol ethoxylates with saturated carbon chain and having HLB higher than 15.5 are Brij 35 (also known as Laureth-23 or HO-(C₂H₄O)₂₃C₁₂H₂₅ or polyoxyethylene lauryl ether (a C12EO23 compound)) or Brij 700 (also known as steareth-100 or polyoxyethylene (100) stearyl ether (C18EO100)). Suitable examples of (b) the class of fatty alcohol ethoxylates with unsaturated carbon chain with HLB higher than 12 are Brij 97 (also known as Oleth-10 or HO-(C₂H₄O)₁₀C₁₈H₃₅ or polyoxyethylene 10 oleyl ether (unsaturated C18EO12)), or Brij 99 (polyoxyethylene (20) oleyl ether (unsaturated C18EO20)). Suitable examples of (c) alkyl phenol ethoxylates with HLB higher than 15 for use in the composition of the invention are Triton X 165, Triton X 305, Triton 405, or Triton X 705. Suitable examples of (d) polyoxyethylene sorbitan alkyl esters with saturated C12 to C16 carbon chain and having HLB higher than 12 and (e) polyoxyethylene sorbitan alkyl esters with unsaturated C18 carbon chain and having HLB higher than 9 are Tween 20, Tween 21, Tween 40, Tween 80, Tween 81 or Tween 85 trioleate. The non-ionic surfactant is included in 0.1 to 5 %, preferably 0.2 to 4 %, more preferably 0.2 to 3 % by weight of the composition. Especially useful non-ionic surfactant is a polyoxyethylene/ polyoxypropylene based surfactant.

The composition of the invention comprises 0.05 to 15 % by weight compound of the formula A: 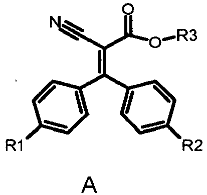 where R1 and R2 are each independently hydrogen or straight or branched chain C1-C30 alkoxy group, where R1 and R2 are not simultaneously hydrogen; and and R3 is a straight or branched chain C1-C30 alkyl group.

R3 is preferably a C2-C20 alkyl group.

The group R1 and/or R2 are preferably straight or branched chain C1-C8 alkoxy group. Most preferably R1 and/or R2 are a methoxy group or a polymer anchored through an alkoxy group.

A highly preferred aspect provides for R1 to be methoxy, R2 to be hydrogen and R3 to be a ethyl hexyl group. In this case the compound is known as ethylhexyl methoxycrylene (EHMC):

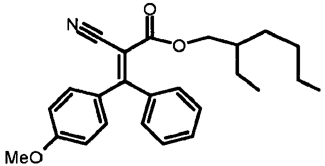

Other useful compound of formula A is when when R1 is allyloxy it is known as ethyl allyloxycrylene:

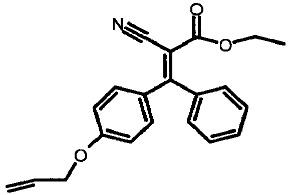

Yet another useful compound of formula A is when R1 is polysiloxane covalently linked via propyloxy (-OCH2CH2CH2-polysiloxane) and the compound is then known as polymeric propyloxycrylene:

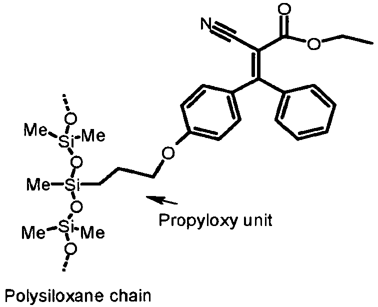

Compound of formula A is preferably present in 0.1 to 10 %, more preferably 0.1 to 4% by weight of the composition. A preferred composition of the invention is one where the weight ratio of dibenzoylmethane derivative to compound of formula A is from 10:1 to 1:10, preferably from 5:1 to 1:5, further more preferably 2:1 to 1:2.

Combinations of UV-A sunscreen (e.g. of the dibenzoylmethane class) and a UV-B sunscreen have been used to get high SPF benefits. But when these two are used together, it is observed that the UV-A compound tends to be unstable. It has been observed that the use of well known photostabilisers like Octocrylene in a composition comprising a UV-A sunscreen and a UV-B sunscreen is not able to provide the high SPF desired when low amounts of sunscreen are used. It has been surprisingly found that use of a compound of formula A in a composition of the invention is not only able to provide the high SPF but enhanced efficacy (by way of stability) of the sunscreens when in use. Without wishing to be bound by theory it is believed that this enhanced stabilization is due to the synergistic effect of the UV sunscreens when used in the composition of the present invention comprising the non-ionic surfactants in combination with the compound of formula A which acts as a singlet excited state quencher to the optimum extent. The desired effect is not observed when other well known stabilizers are used e.g. Octocrylene™ or undecylcrylene dimethicone (Hallbrite form PSF) or Polycrylene™ (Polyester 8 which is a copolymer of adipic acid and neopentyl glycol terminated with cyanodiphenyl propenoic acid), instead of the compound of formula A.

The composition of the invention comprises a cosmetically acceptable base. The cosmetically acceptable bases are such as to have a product in preferably a cream, lotion, gel or emulsion format. A more preferred format is a cream, further more preferably a vanishing cream. Vanishing cream base is one which comprises 5 to 25 %, more preferably 5 to 20 % fatty acid. The base preferably comprises 0.1 to 10 %, more preferably 0.1 to 3 % soap. C₁₂ to C₂₀ fatty acids are especially preferred in vanishing cream bases, further more preferred being C₁₄ to C₁₈ fatty acids. In creams, the fatty acid is preferably substantially a mixture of stearic acid and palmitic acid. Soaps in the vanishing cream base include alkali metal salt of fatty acids, like sodium or potassium salts The soap is preferably the potassium salt of the fatty acid mixture. The fatty acid in vanishing cream base is often prepared using hystric acid which is substantially (generally about 90 to 95 %) a mixture of stearic acid and palmitic acid. Thus, inclusion of hystric acid and its soap to prepare the vanishing cream base is within the scope of the present invention. The composition comprises at least 6%, preferably at least 10 %, more preferably at least 12 % fatty acid. The cosmetically acceptable base is from 10 to 99 %, preferably from 50 to 99 % by weight of the composition. It has been observed that use of such high levels of fatty acid also contributes to the high SPF. The cosmetically acceptable base preferably includes water. Water is preferably included in 35 to 90 %, more preferably 50 to 85 %, further more preferably 50 to 80 % by weight of the composition.

Other useful sun-protective agents e.g. inorganic sun-blocks may be preferably used in the present invention. These include, for example, zinc oxide, iron oxide, silica, such as fumed silica, or titanium dioxide. The total amount of sun block that is preferably incorporated in the composition according to the invention is from 0.1 to 5 % by weight of the composition.

The composition of the invention may additionally comprise a skin lightening agent. The skin lightening agent is preferably chosen from a vitamin B3 compound or its derivative e.g. niacin, nicotinic acid, niacinamide or other well known skin lightening agents e.g. aloe extract, ammonium lactate, azelaic acid, kojic acid, citrate esters, ellagic acid, glycolic acid, green tea extract, hydroquinone, lemon extract, linoleic acid, magnesium ascorbyl phosphate, vitamins like vitamin B6, vitamin B12, vitamin C, vitamin A, a dicarboxylic acid, resorcinol derivatives, hydroxycarboxylic acid like lactic acid and their salts e.g. sodium lactate, and mixtures thereof. Vitamin B3 compound or its derivative e.g. niacin, nicotinic acid, niacinamide are the more preferred skin lightening agent as per the invention, most preferred being niacinamide. Niacinamide, when used, is preferably present in an amount in the range of 0.1 to 10 %, more preferably 0.2 to 5 % by weight of the composition.

The composition according to the invention may also comprise other diluents. The diluents act as a dispersant or carrier for other materials present in the composition, so as to facilitate their distribution when the composition is applied to the skin. Diluents other than water can include liquid or solid emollients, solvents, humectants, thickeners and powders.

The composition of the invention may comprise a conventional deodourant base as the cosmetically acceptable carrier. By a deodorant is meant a product in the stick, roll-on, or propellant medium which is used for personal deodorant benefit e.g. application in the under-arm or any other area which may or may not contain anti-perspirant actives.

Deodorant compositions can generally be in the form of firm solids, soft solids, gels, creams, and liquids and are dispensed using applicators appropriate to the physical characteristics of the composition.

The compositions of the present invention can comprise a wide range of other optional components. The CTFA Cosmetic Ingredient Handbook, Second Edition, 1992, which is incorporated by reference herein in its entirety, describes a wide variety of non-limiting cosmetic and pharmaceutical ingredients commonly used in the skin care industry, which are suitable for use in the compositions of the present invention. Examples include: antioxidants, binders, biological additives, buffering agents, colorants, thickeners, polymers, astringents, fragrance, humectants, opacifying agents, conditioners, exfoliating agents, pH adjusters, preservatives, natural extracts, essential oils, skin sensates, skin soothing agents, and skin healing agents.

The invention is now further described by way of the following non-limiting examples.

### Examples

### Examples 1 to 3: Photostability of the composition of the invention as compared to compositions outside the invention

Photoprotective personal care vanishing cream compositions as shown in table 1 were prepared.

**Table 1**

| Ingredients | Example 1 | Example 2 | Example 3 |
|---|---|---|---|
| Hystric acid | 17.00 | 17.00 | 17.00 |
| KOH (85%) | 0.56 | 0.56 | 0.56 |
| Cetyl alcohol | 0.53 | 0.53 | 0.53 |
| Isopropyl myristate | 1.64 | 1.64 | 1.64 |
| Parsol 1789 | 1.20 | 1.20 | 1.20 |
| Parsol MCX | 2.25 | 2.25 | 2.25 |
| Ethylhexyl methoxycrylene | 1.50 | 0.0 | 0.0 |
| Brij 35 | 2.00 | 2.00 | 2.00 |
| Octocrylene™ | 0.0 | 0.0 | 1.20 |
| Methyl paraben | 0.20 | 0.20 | 0.20 |
| Propyl paraben | 0.10 | 0.10 | 0.10 |
| Phenoxy ethanol | 4.40 | 4.4 | 4.40 |
| Glycerine | 1.00 | 1.00 | 1.00 |
| Disodium EDTA | 0.04 | 0.04 | 0.04 |
| Dimethicone | 0.50 | 0.50 | 0.50 |
| Water | To 100 | To 100 | To 100 |

The compositions (examples 1 to 3) shown in table 1, were applied (∼ 3 mg/cm²) on four clean glass plates to generate thin films of uniform thickness. Out of these, three plates were exposed to Atlas solar simulated radiations (UVA flux, 5.5 mW/cm²). One plate each is removed after 30, 60 and 120 minutes of UV exposure. The fourth plate was kept unexposed which served as a control. Subsequent to completion of the above protocol, all four films of the cream were separately dissolved in suitable HPLC grade methanol. The UVA (Parsol 1789) quantification was done using Perkin Elmer UV/Visible Spectrometer. The absorbance at a scanning range of 200-800 nm was measured for each solution using quartz cuvette and respective blank solutions spectrometer. The data on % Parsol 1789 remaining is shown in table 2:

**Table 2**

| | Example 1 | Example 2 | Example 3 |
|---|---|---|---|
| % UVA remaining after 30 min | 79.70 | 31.90 | 55.16 |
| % UVA remaining after 60 min | 57.23 | 16.69 | 29.74 |
| % UVA remaining after 120 min | 51.98 | 7.60 | 12.95 |

The data in table 2 indicates that the composition as per the invention (example 1) is vastly superior to that of a composition without compound of formula A (example 2) and also vastly superior to a composition using another well known sunscreen stabilizer (Octocrylene™).

### Examples 4 to 6

Various composition as shown in table 1 were prepared except that the amount of Ethyl Hexyl Methoxycrylene (EHMC) and Brij 35 were varied with the level of water being raised accordingly. Invitro-SPF was measured using the Optometrics 290S instrument model in accordance with standard protocol ISO/WD 24445. The substrate used was a 8 cm Transpore tape (3M Company). The sample was applied at 2 mg/cm². The SPF as measured is shown in table 3.

**Table 3**

| Composition | 4 | 5 | 6 |
|---|---|---|---|
| EHMC | 0.765 | 0.765 | 1.53 |
| Brij 35 | - | 1 | 1 |
| SPF | 8.76 | 22.65 | 23.5 |

The data in table 3 indicates that use of a non-ionic surfactant and a ethyl hexyl methoxycrylene provides for vast improvement in sun protection in a sunscreen composition comprising a dibenzoyl methane sunscreen and an oil soluble UV-B sunscreen.

### Example 7: Photoprotective hair care composition with EHMC (not according to the invention)

A hair care formulation was made in the form of a hair styling gel. Aristoflex AVC (an ammonium acryloyldimethyltaurate/vinyl pyrrolidone copolymer and Brij -35 were dissolved in water using a homogenizer. Parsol 1789™, Parsol MCX and EHMC were separately dissolved in propylene carbonate. The above two mixtures were then mixed and homogenized by using a high speed homogenizer. The composition is shown in table 4.

**Table 4**

| Ingredients | Percentage |
|---|---|
| Aristoflex AVC (cationic polymer) | 1.20 |
| Propylene carbonate (oil phase) | 9.00 |
| Parsol 1789™ | 1.20 |
| Parsol MCX | 2.25 |
| EHMC | 1.20 |
| Brij 35 | 1.00 |
| Methyl paraben | 0.20 |
| Propyl paraben | 0.10 |
| Water | To 100 |

The present invention thus provides for high SPF photo-protective sunscreen composition while ensuring prolonged efficacy of the UV-A sunscreen used. All this is achieved using low amounts of sunscreen agents thereby keeping costs low.

## Claims

1. A sunscreen composition comprising,
a) 0.01 to 10 % by weight dibenzoylmethane or its derivatives selected from 4-tert-butyl-4'-methoxydibenzoylmethane, 2-methyldibenzoylmethane, 4-methyl-dibenzoyl-ethane, 4-isopropyldibenzoyl-methane, 4-tert-butyldibenzoylmethane, 2,4-dimethyldibenzoy(methane, 2,5-dimethyldibenzoylmethane, 4,4'-diisopropyl-dlbenzoylmethane, 2-methyl-5-isopropyl-4'-methoxydibenzoylmethane, 2-methyl-5-tert-butyl-4'-methoxy-dibenzoyl methane, 2,4-dimethyl-4'-methoxy dibenzoylmethane or 2,6-dimethyl-4-tert-butyl-4'-methoxy-dibenzoylmethane;
b) 0.01 to 10 % by weight an oil soluble UV-B organic sunscreen selected from the group consisting of cinnamic acid, salicylic acid, diphenyl acrylic acid and derivatives thereof,
c) 0.01 to 10 % by weight a non-ionic surfactant;
d) 0.05 to 15 % by weight compound of formula; and 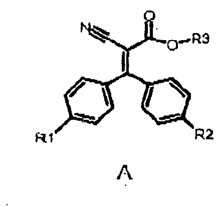
where R1 and R2 are each independently hydrogen or straight or branched chain C1-C30 alkoxy group, where R1 and R2 are not simultaneously hydrogen;
and R3 is a straight or branched chain C1-C30 alkyl group; and
e) from 10 to 99 % by weight of a cosmetically acceptable base comprising 5-25% fatty acid by weight of the cosmetically acceptable base, wherein the composition comprises at least 6 % by weight of fatty acid.

2. A composition as claimed in claim 1 wherein said non-ionic surfactant has an HLB value of at least 9.

3. A composition as claimed in claim 2 wherein said non-ionic surfactant is selected from the class comprising
(a) fatty alcohol ethoxylates with saturated carbon chain and having HLB higher than 15.5; or
(b) fatty alcohol ethoxylates with unsaturated carbon chain with HLB higher than 12
(c) alkyl phenol ethoxylates having HLB higher than 15;
(d) polyoxyethylene sorbitan alkyl esters with saturated C12 to C16 carbon chain and having HLB higher than 12; or
(e) polyoxyethylene sorbitan alkyl esters with unsaturated C18 carbon chain and having HLB higher than 9.

4. A composition as claimed in any claim one of the preceding claims, wherein said dibenzoylmethane derivative is 4-tert-butyl-4'-methoxydibenzoylmethane.

5. A composition as claimed in any one of the preceding claim, wherein said dibenzoylmethane or its derivative is present from 0.1 to 5 % by weight.

6. A composition as claimed in any one of the preceding claims wherein said oil-soluble UV-B organic sunscreen is present in 0.1 to 5 % by weight of the composition.

7. A composition as claimed in any one of the preceding claims, wherein said oil-soluble UV-B sunscreen is 2-ethyl-hexyl-4-methoxy cinnamate.

8. A composition as claimed in any one of the preceding claims comprising less than 10 % by weight total organic sunscreens

9. A composition as claimed in any one of the preceding claims wherein the cosmetically acceptable base comprises 0.1 to 10% by weight soap.

## Patentansprüche

1. Sonnenschutzzusammensetzung,
die Folgendes aufweist:
a) 0,01 bis 10 Gew.-% Dibenzoylmethan oder dessen Derivate, die ausgewählt sind aus 4-tert.-Butyl-4'-methoxydibenzoylmethan, 2-Methyldibenzoylmethan, 4-Methyldibenzoylethan, 4-Isopropyldibenzoylmethan, 4-tert.-Butyldibenzoylmethan, 2,4-Dimethyldibenzoylmethan, 2,5-Dimethyldibenzoylmethan, 4,4'-Diisopropyldibenzoylmethan, 2-Methyl-5-isopropyl-4'-methoxydibenzoylmethan, 2-Methyl-5-tert.-butyl-4'-methoxydibenzoylmethan, 2,4-Dimethyl-4'-methoxydibenzoylmethan oder 2,6-Dimethyl-4-tert.-butyl-4'-methoxydibenzoylmethan;
b) 0,01 bis 10 Gew.-% eines öllöslichen organischen UV-B-Sonnenschutzmittels, das aus der Gruppe ausgewählt ist, die aus Zimtsäure, Salicylsäure, Diphenylacrylsäure und Derivaten davon besteht;
c) 0,01 bis 10 Gew.-% eines nichtionischen Tensids;
d) 0,05 bis 15 Gew.-% einer Verbindung der folgenden Formel 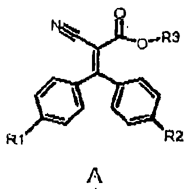
worin R1 und R2 jeweils unabhängig voneinander Wasserstoff oder eine geradkettige oder verzweigte C₁-C₃₀-Alkoxygruppe sind, wobei R1 und R2 nicht gleichzeitig Wasserstoff sind; und
R3 eine geradkettige oder verzweigte C₁-C₃₀-Alkylgruppe ist; und
e) 10 bis 99 Gew.-% eines kosmetisch akzeptablen Trägers, der 5 bis 25 % Fettsäure aufweist, und zwar auf das Gewicht des kosmetisch akzeptablen Trägers bezogen,
wobei die Zusammensetzung mindestens 6 Gew.-% Fettsäure aufweist.

2. Zusammensetzung nach Anspruch 1,
wobei das nichtionische Tensid einen HLB-Wert von mindestens 9 aufweist.

3. Zusammensetzung nach Anspruch 2,
wobei das nichtionische Tensid aus der Klasse ausgewählt ist, die Folgendes aufweist:
(a) Fettalkoholethoxylate mit einer gesättigten Kohlenstoffkette und einem HLB-Wert von mehr als 15,5; oder
(b) Fettalkoholethyoxylate mit einer ungesättigten Kohlenstoffkette mit einem HLB-Wert von mehr als 12;
(c) Alkylphenolethoxylate mit einem HLB-Wert von mehr als 15;
(d) Polyoxyethylensorbitanalkylester mit einer gesättigten C₁₂-C₁₆-Kohlenstoffkette und einem HLB-Wert von mehr als 12; oder
(e) Polyoxyethylensorbitanalkylester mit einer ungesättigten C₁₈-Kohlenstoffkette und einem HLB-Wert von mehr als 9.

4. Zusammensetzung nach einem der vorstehenden Ansprüche,
wobei das Dibenzoylmethan-Derivat 4-tert.-Butyl-4'-methoxydibenzoylmethan ist.

5. Zusammensetzung nach einem der vorstehenden Ansprüche,
wobei das Dibenzoylmethan oder dessen Derivat mit 0,1 bis 5 Gew.-% vorliegt.

6. Zusammensetzung nach einem der vorstehenden Ansprüche,
wobei das öllösliche organische UV-B-Sonnenschutzmittel mit 0,1 bis 5 Gew.-% der Zusammensetzung vorliegt.

7. Zusammensetzung nach einem der vorstehenden Ansprüche,
wobei öllösliche UV-B-Sonnenschutzmittel 2-Ethylhexyl-4-methoxycinnamat ist.

8. Zusammensetzung nach einem der vorstehenden Ansprüche,
die insgesamt weniger als 10 Gew.-% organische Sonnenschutzmittel aufweist.

9. Zusammensetzung nach einem der vorstehenden Ansprüche,
wobei der kosmetisch akzeptable Träger 0,1 bis 10 Gew.-% Seife aufweist.

## Revendications

1. Composition d'écran solaire comprenant,
a) de 0,01 à 10 % en poids de dibenzoylméthane ou de ses dérivés choisis parmi le 4-tert-butyl-4'-méthoxydibenzoylméthane, le 2-méthyldibenzoylméthane, le 4-méthyl-dibenzoyl-éthane, le 4-isopropyldibenzoyl-méthane, le 4-tert-butyldibenzoylméthane, le 2,4-diméthyldibenzoylméthane, le 2,5-diméthyldibenzoylméthane, le 4,4'-diisopropyl-dibenzoylméthane, le 2-méthyl-5-isopropyl-4'-méthoxy-dibenzoylméthane, le 2-méthyl-5-tert-butyl-4'-méthoxydibenzoylméthane, le 2,4-diméthyl-4'-méthoxydibenzoylméthane ou le 2,6-diméthyl-4-tert-butyl-4'-méthoxy-dibenzoylméthane ;
b) de 0,01 à 10 % en poids d'un écran solaire organique aux UV-B soluble dans l'huile choisi dans le groupe constitué d'acide cinnamique, d'acide salicylique, d'acide diphénylacrylique et de dérivés de ceux-ci,
c) de 0,01 à 10 % en poids d'un tensioactif non ionique ;
d) de 0,05 à 15 % en poids de composé de formule ; et 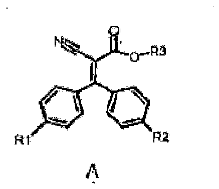
où R1 et R2 sont chacun indépendamment l'hydrogène ou un groupe alcoxy en C1-C30 à chaîne linéaire ou ramifié, où R1 et R2 ne sont pas simultanément l'hydrogène ;
et R3 est un groupe alkyle en C1-C30 à chaîne linéaire ou ramifié ; et
e) de 10 à 99 % en poids d'une base cosmétiquement acceptable comprenant 5-25 % d'acide gras en poids de la base cosmétiquement acceptable, dans laquelle la composition comprend au moins 6 % en poids d'acide gras.

2. Composition selon la revendication 1, dans laquelle ledit tensioactif non ionique présente une valeur HLB d'au moins 9.

3. Composition selon la revendication 2, dans laquelle ledit tensioactif non ionique est choisi dans la classe comprenant
(a) des éthoxylates d'alcools gras avec une chaîne carbonée saturée et présentant un HLB supérieur à 15,5 ; ou
(b) des éthoxylates d'alcools gras avec une chaîne carbonée insaturée avec un HLB supérieur à 12
(c) des éthoxylates d'alkylphénol présentant un HLB supérieur à 15;
(d) des esters alkyliques de polyoxyéthylène sorbitane avec une chaîne carbonée en C12 à C16 saturée et présentant un HLB supérieur à 12 ; ou
(e) des esters alkyliques de polyoxyéthylène sorbitane avec une chaîne carbonée en C18 insaturée et présentant un HLB supérieur à 9.

4. Composition selon l'une quelconque des revendications précédentes, dans laquelle ledit dérivé de dibenzoylméthane est le 4-tert-butyl-4'-diméthoxydibenzoylméthane.

5. Composition selon l'une quelconque des revendications précédentes, dans laquelle ledit dibenzoylméthane ou son dérivé est présent à de 0,1 à 5 % en poids.

6. Composition selon l'une quelconque des revendications précédentes, dans laquelle ledit écran solaire organique aux UV-B soluble dans l'huile est présent à de 0,1 à 5 % en poids de la composition.

7. Composition selon l'une quelconque des revendications précédentes, dans laquelle ledit écran solaire aux UV-B soluble dans l'huile est le 2-éthyl-hexyl-4-méthoxycinnamate.

8. Composition selon l'une quelconque des revendications précédentes comprenant moins de 10 % en poids d'écrans solaires organiques au total.

9. Composition selon l'une quelconque des revendications précédentes, dans laquelle la base cosmétiquement acceptable comprend de 0,1 à 10 % en poids de savon.
